# EUROPEAN PATENT SPECIFICATION

(11) **EP 4 058 099 B1**
(45) Date of publication and mention of the grant of the patent: **31.01.2024**
(21) Application number: 20821379.3
(22) Date of filing: 12.11.2020
(51) Int. Cl.: A61M 1/36

(54) **EQUIPMENT FOR THE CONTROL OF BIOMEDICAL DEVICES DURING EXTRACORPOREAL CIRCULATION AND RELATED SYSTEM**
VORRICHTUNG ZUR STEUERUNG VON BIOMEDIZINISCHEN VORRICHTUNGEN WÄHREND DER EXTRAKORPORALEN ZIRKULATION UND ENTSPRECHENDES SYSTEM
ÉQUIPEMENT POUR LE CONTRÔLE DE DISPOSITIFS BIOMÉDICAUX PENDANT UNE CIRCULATION EXTRACORPORELLE ET SYSTÈME ASSOCIÉ

(30) Priority: 12.11.2019 IT 201900021012
(43) Date of publication of application: 21.09.2022
(73) Proprietor: Eurosets S.r.l., 41036 Medolla (MO) (IT)
(72) Inventor: GHELLI, Nicola, 41036 Medolla (MO) (IT); FONTANILI, Paolo, 41036 Medolla (MO) (IT); CORBELLI, Marco, 41036 Medolla (MO) (IT)
(74) Representative: Zoli, Filippo
(86) International application number: PCT/IB2020/060654
(87) International publication number: WO 2021/094971

(56) References cited:
- DE-A1-102018 106 906
- US-B1- 6 808 508
- US-B1- 6 868 309

## Description

### Technical Field

The present invention relates to a piece of equipment for the control of biomedical devices during extracorporeal circulation and a related system.

### Background Art

As it is well known, for certain surgical operations, during which the functions of the patient's heart are temporarily suspended, extracorporeal blood circuits are created that involve the use of biomedical devices, such as e.g. the so-called "heart-lung" machines.

The heart-lung machines comprise a series of devices including a filtration device (also called "venous reserve") adapted to filter the blood coming from the patient, a heat exchanger adapted to regulate the temperature of the blood coming out of the filtration device, and an oxygenator adapted to provide the correct supply of oxygen to the blood intended to be fed back into the patient. In particular, the blood arriving from the patient is conveyed to the oxygenator by means of a relevant pumping unit, which generally comprises a centrifugal pump of the dragging or magnetic levitation type, i.e. provided with a rotor element adapted to convey the incoming blood to the oxygenator as a result of the rotation thereof and with a stator element adapted to control the rotor element in rotation.

During extracorporeal circulation, a number of parameters must be monitored and controlled, including the number of revolutions of the rotor element and the flow rate of the blood conveyed to the oxygenator.

In this regard, the equipment of known type comprises control means configured to control the biomedical device and to monitor the operation thereof.

This piece of equipment is nowadays particularly used also in the extra-hospital field since the extracorporeal circulation is also applied in emergency situations, e.g. in roadside assistance, during which there is the need to adequately support the various devices in a dynamic and flexible manner.

The equipment of known type for the control of biomedical devices and the relevant systems do have some drawbacks.

In particular, they are significantly affected by any malfunction of the control means.

In fact, as a result of a malfunction of the control means, the equipment of known type must be promptly replaced with a piece of equipment which is functioning.

A replacing piece of equipment is however not always available and such replacement takes a not negligible amount of time during which the patient's safety is put at risk.

This drawback makes known types of equipment particularly unreliable and risky.

Other equipment for the control of biomedical devices during extracorporeal circulation and related system are known from DE 10 2018 106906 A1, US 6 868 309 B1 and US 6 808 508 B1.

### Description of the Invention

The invention is defined by the features of independent claim 1.

The main aim of the present invention is to devise a piece of equipment and a related system that allow ensuring the correct operation of the biomedical device, of the equipment itself and of the relevant system also as a result of a malfunction of the control means.

Another object of the present invention is to devise a piece of equipment for the control of biomedical devices during extracorporeal circulation and a related system that allow overcoming the above mentioned drawbacks of the prior art within a simple, rational, easy, effective to use and low cost solution.

The objects set out above are achieved by the present piece of equipment for the control of biomedical devices during extracorporeal circulation having the characteristics of claim 1.

The objects set out above are achieved by the present system for the control of biomedical devices during extracorporeal circulation having the characteristics of claim 7.

### Brief Description of the Drawings

Other characteristics and advantages of the present invention will be more evident from the description of a preferred, but not exclusive, embodiment of a piece of equipment for the control of biomedical devices during the extracorporeal circulation and of a related system, illustrated by way of an indicative, yet non-limiting example, in the attached tables of drawings in which:

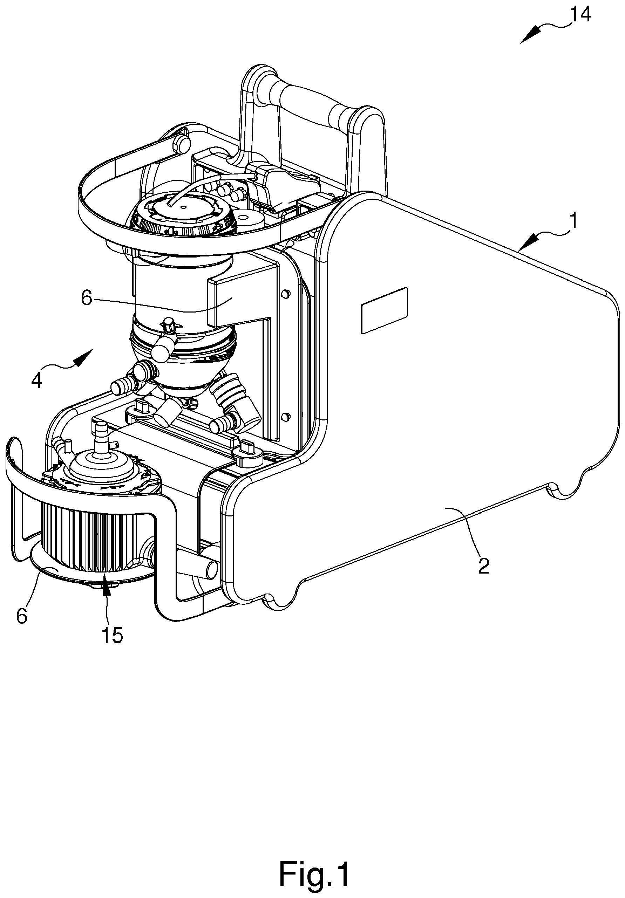

Figure 1 is a schematic axonometric view of the system according to the invention;

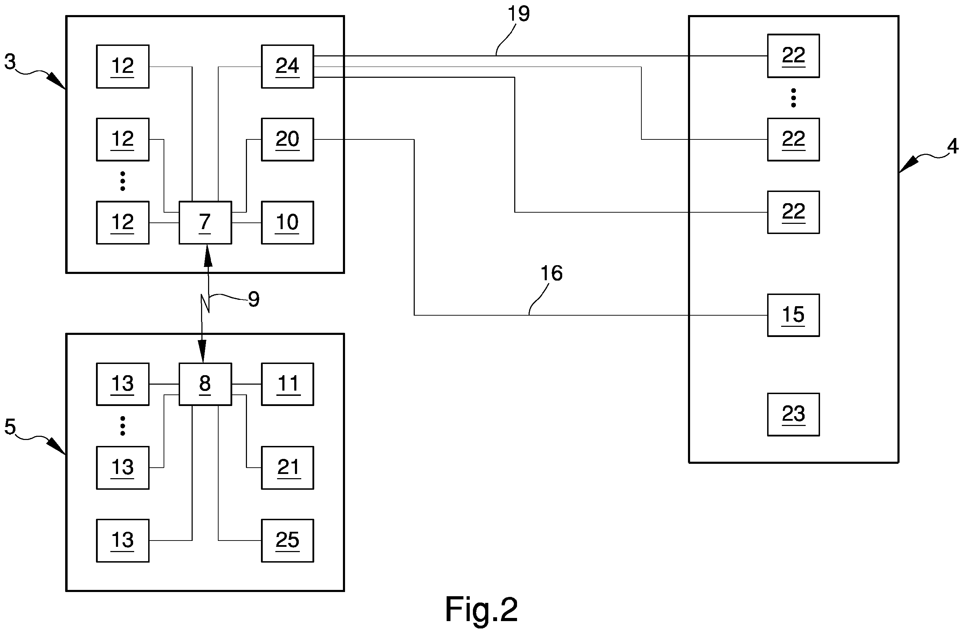

Figure 2 is a block diagram of the system according to the invention in a first working configuration;

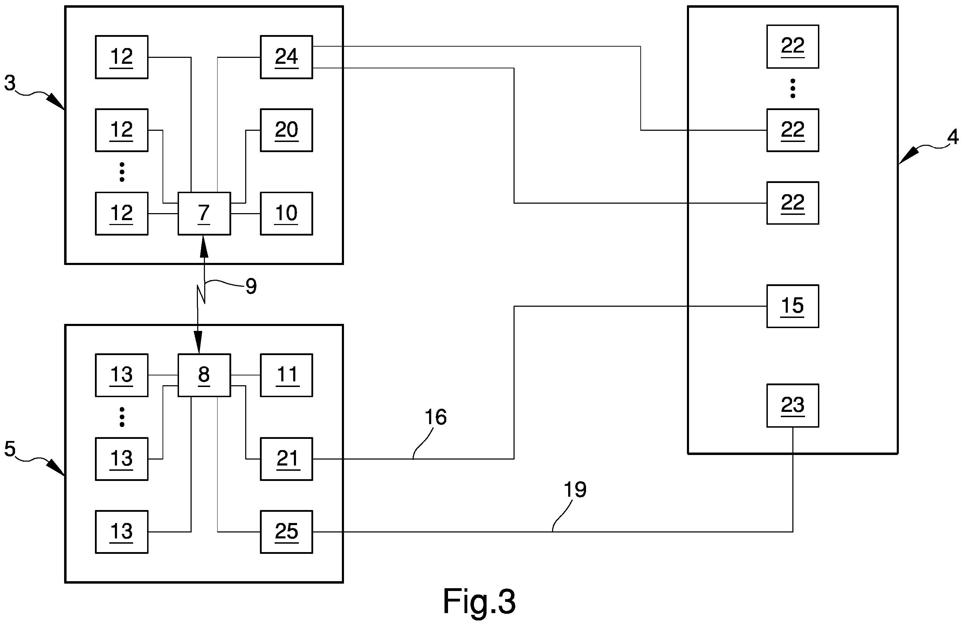

Figure 3 is a block diagram of the system according to the invention in a second working configuration.

### Embodiments of the Invention

With particular reference to these figures, reference numeral 1 globally indicates a piece of equipment for the control of biomedical devices during the extracorporeal circulation.

The piece of equipment 1 for the control of biomedical devices during the extracorporeal circulation comprises:
- at least one basic structure 2;
- control means 3, 5, 9, 10, 11 of the piece of equipment 1 assembled on the basic structure 2 and provided with at least one control unit 3 operationally connectable to at least one biomedical device 4 for the extracorporeal circulation and configured to control the latter.

Advantageously, the control means 3, 5, 9, 10, 11 comprise at least one emergency control unit 5, operationally connectable to the biomedical device 4 and configured to control the latter as a result of a malfunction of the control unit 3.

This way, if the control unit 3 stops operating properly, the functionality of the biomedical device 4 is ensured by the emergency control unit 5.

Conveniently, the basic structure 2 comprises support means 6 of the biomedical device 4 adapted to fasten the latter to the same basic structure 2 in a removable manner.

Conveniently, the control means 3, 5, 9, 10, 11 comprise communication means 9 between the control unit 3 and the emergency control unit 5.

According to the invention, the control unit 3 and the emergency control unit 5 are configured to check the malfunction of the other.

This way, any malfunction of the control unit 3 is immediately detected by the emergency control unit 5.

In addition, this device makes it possible to immediately identify any malfunction of the emergency control unit 5 during the correct operation of the control unit 3.

This way, it is possible to intervene promptly with the repair of the emergency control unit 5 and to prevent the emergency control unit 5 from malfunctioning and therefore being unusable as a result of the malfunction of the control unit 3, putting the patient's safety at risk.

In particular, the control unit 3 comprises at least one central unit 7 for the management of the same control unit and configured to check the malfunction of the emergency control unit 5.

Similarly, the emergency control unit 5 comprises at least one central emergency unit 8 for the control of the same emergency control unit and configured to check the malfunction of the control unit 3.

Therefore, according to the invention, the communication means 9 are operationally connected to the central unit 7 and to the central emergency unit 8 in order to allow these to check the malfunction of the emergency control unit 5 and of the control unit 3 respectively.

Preferably, the central unit 7 is an electronic device of the type e.g. of a microprocessor, microcontroller, PLC and the like.

Similarly, the central emergency unit 8 is an electronic device of the type e.g. of a microprocessor, microcontroller, PLC, control unit and the like.

In addition, the communication means 9 are preferably of the type of a low-voltage two-way electronic link for signal exchange.

Advantageously, the control means 3, 5, 9, 10, 11 comprise signaling means 10, 11 for signaling the malfunction of at least one of either the control unit 3 or the emergency control unit 5.

Preferably, the control unit 3 comprises at least one signaling unit 10 configured to notify a user of the malfunction of the emergency control unit 5.

Likewise, the emergency control unit 5 preferably comprises at least one emergency signaling unit 11 configured to notify a user of the malfunction of the control unit 3.

In addition, preferably, the signaling unit 10 is operationally connected to the central unit 7.

Likewise, the emergency signaling unit 11 is operationally connected to the central emergency unit 8.

Conveniently, the control unit 3 comprises at least one input/output device 12 configured to check/monitor the biomedical device 4.

In particular, the input/output device 12 is of the type selected from the list comprising a keyboard, a screen, a USB socket, a code reader, an electronic switch.

Preferably, the input/output device 12 is operationally connected to the central unit 7.

In addition, the control unit 3 comprises a plurality of input/output devices 12. In particular, according to the invention, the control unit 3 comprises an input/output device 12 of each type included in the above mentioned list. Conveniently, the emergency control unit 5 comprises at least one emergency input/output device 13 of the type selected from the list comprising a keyboard, a screen, a USB socket, a code reader, an electronic switch.

Preferably, the emergency input/output device 13 is operationally connected to the central emergency unit 8.

Preferably, the control unit 3 comprises a plurality of emergency input/output devices 13.

In particular, according to the invention, the emergency control unit 5 comprises an emergency input/output device 13 of each type included in the above mentioned list.

This way, the control unit 3 and the emergency control unit 5 have the same input/output devices for the correct management of the same equipment and of the biomedical device 4.

Conveniently, the equipment 1 comprises connection means 16, 19 of the biomedical device 4 to one of either the control unit 3 or the emergency control unit 5.

In particular, the connection means 16, 19 are connectable in a removable manner between at least a first operating configuration, wherein they operationally connect the biomedical device 4 to the control unit 3, and at least a second operating configuration, wherein they operationally connect the biomedical device 4 to the emergency control unit 5.

This way, as a result of a malfunction of the control unit 3, the biomedical device 4 is controlled by the emergency control unit 5, which ensures the correct operation of the biomedical device 4.

Advantageously, the connection means 16, 19 are operationally connectable to a plurality of components 15, 22, 23 of the biomedical device 4.

In particular, the connection means 16, 19 connect, in a removable manner, at least one component 15, 22, 23 to the control unit 3 and to the emergency control unit 5 in the first and in the second operating configuration, respectively. This way, the connection means 16, 19 allow each of the components 15, 22, 23 to be connected to the emergency control unit 5 in the event of a malfunction in the control unit 3.

This way, the equipment 1 allows ensuring the operation of the biomedical devices 4 provided with a plurality of components 15, 22, 23 which are essential to ensure proper extracorporeal circulation.

The present invention also relates to a system for extracorporeal circulation globally indicated with reference number 14.

The system 14 for extracorporeal circulation comprises:
- at least one biomedical device 4 for the extracorporeal circulation;
- at least one piece of equipment 1 having one or more of the characteristics of the equipment 1 described above.

Advantageously, the biomedical device 4 comprises at least one pumping motor-driven unit 15 for extracorporeal circulation.

In addition, the connection means 16, 19 comprise at least one connecting line 16 which operationally connects the motor-driven unit 15 to the control unit 3 and to the emergency control unit 5 in the first and in the second operating configuration, respectively.

Preferably, the connecting line 16 can be manually connected/disconnected by a user who, as a result of a malfunction of the control unit 3, disconnects the connecting line 16 from the latter and connects it to the emergency control unit 5.

Furthermore, the connecting line 16 is of the type of an electronic connection device provided with connectors such as, e.g, an electric cable or the like. Advantageously, the control unit 3 comprises at least one drive unit 20 of the motor-driven unit 15 and the emergency control unit 5 comprises at least one emergency drive unit 21 of the motor-driven unit 15.

This way, the connecting line 16 operationally connects the motor-driven unit 15 to the drive unit 20 and to the emergency drive unit 21 in the first operating configuration and in the second operating configuration, respectively.

In particular, the drive unit 20 is operationally connected to the central unit 7. Similarly, the emergency drive unit 21 is operationally connected to the central emergency unit 8.

Conveniently, the biomedical device 4 comprises at least one monitoring unit 22, 23 of the extracorporeal circulation provided with at least one sensor device 22 selected from the list comprising a flow meter, pressure sensor, level sensor, in-line air detector, venous probe, arterial probe.

In addition, the connection means 16, 19 comprise at least one linking line 19 which operationally connects the sensor device 22 to the control unit 3 and to the emergency control unit 5 in the first operating configuration and in the second operating configuration, respectively.

Conveniently, the linking line 19 can be manually connected/disconnected by a user, who, as a result of a malfunction of the control unit 3, disconnects the linking line 19 from the latter and connects it to the emergency control unit 5. This way, as a result of a malfunction of the control unit 3, the sensor device 22 is controlled by the emergency control unit 5, which uses the data collected by this sensor to ensure the correct extracorporeal circulation.

Preferably, the sensor device 22 is of the type of a flow meter.

Conveniently, the monitoring unit 22, 23 comprises a plurality of sensor devices 22.

Preferably, the monitoring unit 22, 23 comprises at least one sensor device 22 for each type included in the list comprising a pressure sensor, level sensor, in-line air detector, venous probe, arterial probe.

In addition, preferably, the monitoring unit 22, 23 comprises three sensor devices 22 of the type of pressure sensors.

Advantageously, at least one of the sensor devices 22 is operationally connected to the control unit 3.

In particular, according to the invention, all the sensor devices 22 with the exception of the flow meter, are operationally connected in a fixed manner to the control unit 3.

In other words, according to the invention, only the sensor device 22 of the type of a flow meter can be connected in a removable manner to one of either the control unit 3 or the emergency control unit 5 by interposition of the linking line 19. On the contrary, the other sensor devices 22 are operationally permanently connected to the control unit 3, i.e. there is no provision for these sensors to be disconnected from the latter.

Further embodiments of the system 14 cannot however be ruled out wherein the connection means 16, 19 comprise a plurality of linking lines 19, each adapted to connect a respective sensor device 22 to the control unit 3 and to the emergency control unit 5 in the first operating configuration and in the second operating configuration, respectively.

Conveniently, the monitoring unit 22, 23 comprises at least one emergency sensor device 23 selected from the list comprising a flow meter, pressure sensor, level sensor, in-line air detector, venous probe, arterial probe.

In addition, the linking line 19 operationally connects the control unit 3 to the sensor device 22 in the first operating configuration, and operationally connects the emergency control unit 5 to one of either the sensor device 22 or the emergency sensor device 23 in the second operating configuration.

Preferably, the emergency sensor device 23 is a flow meter.

According to the embodiment of the system 14 shown in the figures, in the first operating configuration the linking line 19 operationally connects the control unit 3 to the sensor device 22 and, in the second operating configuration, the linking line 19 operationally connects the emergency sensor device 23 to the emergency control unit 5.

Further embodiments of the system 14 cannot however be ruled out wherein, in the first operating configuration, the linking line 19 operationally connects the control unit 3 to the sensor device 22 and, in the second operating configuration, the linking line 19 connects the same sensor device to the emergency control unit 5.

In other words, as a result of a malfunction of the control unit 3, the linking line 19 must be disconnected from the control unit 3 in order to be connected to the emergency control unit 5 and connect the latter to the sensor device 22 previously connected to the control unit 3.

In addition, further embodiments of the system 14 cannot be ruled out in which the monitoring unit 22, 23 comprises a plurality of emergency sensor devices 23. For example, embodiments of the system 14 cannot be ruled out wherein the monitoring unit 22, 23 comprises one emergency sensor device 23 for each type listed above, or wherein the monitoring unit 22, 23 comprises emergency sensor devices 23 in the same number and of the same type as the sensor devices 22. Advantageously, the control unit 3 comprises at least one check unit 24 of the monitoring unit 22, 23.

In addition, the emergency control unit 5 comprises at least one emergency check unit 25 of the monitoring unit 22, 23.

This way, the linking line 19 operationally connects the monitoring unit 22, 23 to the drive unit 20 and to the emergency drive unit 21 in the first operating configuration and in the second operating configuration, respectively.

In particular, according to the invention, the sensor devices 22 other than the flow meter are operationally permanently connected to the check unit 24.

It has, in practice, been ascertained that the described invention achieves the intended objects.

In particular, the emergency control unit ensures the correct operation of the equipment and of the related system even in the event of a malfunction of the control unit.

In addition, the communication means make it possible to identify in advance any malfunction of the emergency control unit, thus reducing the risk of simultaneous malfunction of the control unit and of the emergency control unit.

## Claims

1. Equipment (1) for the control of biomedical devices during extracorporeal circulation, comprising:
- at least one basic structure (2);
- control means (3, 5, 9, 10, 11) assembled on said basic structure (2) and provided with at least one control unit (3) operationally connectable to at least one biomedical device (4) for extracorporeal circulation and configured to control it;
wherein said control means (3, 5, 9, 10, 11) comprise at least one emergency control unit (5), operationally connectable to said biomedical device (4) and configured to control the latter following a malfunction of said control unit (3);
wherein said control means (3, 5, 9, 10, 11) comprise communication means (9) between said control unit (3) and said emergency control unit (5), said emergency control unit (5) being configured to check the malfunction of said control unit (3) and **characterized in that** said control unit (3) is configured to check the malfunction of said emergency control unit (5) during the correct operation of said control unit (3).

2. Equipment (1) according to claim 1, **characterized by** the fact that said basic structure (2) comprises support means (6) of said biomedical device (4) adapted to fasten the latter to the same basic structure in a removable manner.

3. Equipment (1) according to one or more of the preceding claims, **characterized by** the fact that said control means (3, 5, 9, 10, 11) comprise at least one input/output device (12) configured to check/monitor said biomedical device (4), said input/output device (12) being of the type selected from the list comprising a keyboard, a screen, a USB socket, a code reader, an electronic switch.

4. Equipment (1) according to one or more of the preceding claims, **characterized by** the fact that said emergency control unit (5) comprises at least one emergency input/output device (13) configured to check/monitor said biomedical device (4), said emergency input/output device (13) being of the type selected from the list comprising a keyboard, a screen, a USB socket, a code reader, an electronic switch.

5. Equipment (1) according to one or more of the preceding claims, **characterized by** the fact that it comprises connection means (16, 19) of said biomedical device (4) to one of either said control unit (3) and said emergency control unit (5), said connection means (16, 19) being connectable in a removable manner between at least a first operating configuration, wherein they operationally connect said biomedical device (4) to said control unit (3), and at least a second operating configuration, wherein they operationally connect said biomedical device (4) to said emergency control unit (5).

6. Equipment (1) according to one or more of the preceding claims, **characterized by** the fact that said connection means (16, 19) are operationally connectable to a plurality of components (15, 22, 23) of said biomedical device (4), said connection means (16, 19) connecting in a removable manner at least one component (15, 22, 23) to said control unit (3) and to said emergency control unit (5) in said first and in said second operating configuration, respectively.

7. System (14) for the control of biomedical devices during extracorporeal circulation, comprising:
- at least one biomedical device (4) for extracorporeal circulation;
- at least one piece of equipment (1) according to one or more of claims 1 to 6.

8. System (14) according to claim 7, **characterized by** the fact that:
- said biomedical device (4) comprises at least one pumping motor-driven unit (15) for extracorporeal circulation;
- said connection means (16, 19) comprise at least one connecting line (16) which operationally connects said motor-driven unit (15) to said control unit (3) and to said emergency control unit (5) in said first and in said second operating configuration respectively.

9. System (14) according to one or more of claims 7 to 8, **characterized by** the fact that:
- said biomedical device (4) comprises at least one monitoring unit (22, 23) of the extracorporeal circulation provided with at least one sensor device (22) selected from the list comprising a flow meter, pressure sensor, level sensor, in-line air detector, venous probe, arterial probe;
- said connection means (16, 19) comprise at least one linking line (19) which operationally connects the sensor device (22) to said control unit (3) and to said emergency control unit (5) in said first and in said second operating configuration, respectively.

10. System (14) according to claim 9, **characterized by** the fact that said monitoring unit (22, 23) comprises a plurality of said sensor devices (22).

11. System (14) according to claim 9, **characterized by** the fact that:
- said monitoring unit (22, 23) comprises at least one emergency sensor device (23) selected from the list comprising a flow meter, pressure sensor, level sensor, in-line air detector, venous probe, arterial probe;
- said linking line (19) operationally connects said control unit (3) to said sensor device (22) in said first operating configuration, and operationally connects said emergency control unit (5) to one of either said sensor device (22) or said emergency sensor device (23) in said second operating configuration.

## Patentansprüche

1. Ausrüstung (1) für die Steuerung von biomedizinischen Geräten während des extrakorporalen Kreislaufs, umfassend:
- mindestens eine Grundstruktur (2);
- Steuermittel (3, 5, 9, 10, 11), die an der Grundstruktur (2) angeordnet und mit mindestens einer Steuereinheit (3) versehen sind, die in Wirkverbindung mit mindestens einem biomedizinischen Gerät (4) für den extrakorporalen Kreislauf gebracht werden kann und zu dessen Steuerung ausgebildet ist;
wobei die Steuermittel (3, 5, 9, 10, 11) mindestens eine Notfall-Steuereinheit (5) umfassen, die in Wirkverbindung mit dem biomedizinischen Gerät (4) gebracht werden kann und dazu ausgebildet ist, letzteres nach einer Fehlfunktion der Steuereinheit (3) zu steuern;
wobei die Steuerungsmittel (3, 5, 9, 10, 11) Kommunikationsmittel (9) zwischen der Steuereinheit (3) und der Notfall-Steuereinheit (5) umfassen, wobei die Notfall-Steuereinheit (5) dazu ausgebildet ist, die Fehlfunktion der Steuereinheit (3) zu überprüfen,
und **dadurch gekennzeichnet, dass** die Steuereinheit (3) dazu ausgebildet ist, die Fehlfunktion der Notfall-Steuereinheit (5) während des ordnungsgemäßen Betriebs der Steuereinheit (3) zu überprüfen.

2. Ausrüstung (1) nach Anspruch 1, **dadurch gekennzeichnet, dass** die Grundstruktur (2) Stützmittel (6) des biomedizinischen Geräts (4) umfasst, die dazu ausgebildet sind, das letztere auf abnehmbare Weise an der gleichen Grundstruktur zu befestigen.

3. Ausrüstung (1) nach einem oder mehreren der vorhergehenden Ansprüche, **dadurch gekennzeichnet, dass** die Steuermittel (3, 5, 9, 10, 11) mindestens eine Eingabe-/Ausgabevorrichtung (12) umfassen, die zur Kontrolle/Überwachung des biomedizinischen Geräts (4) ausgebildet ist, wobei die Eingabe-/Ausgabevorrichtung (12) vom Typ einer Tastatur, eines Bildschirms, einer USB-Buchse, eines Codelesers oder eines elektronischen Schalters ist.

4. Ausrüstung (1) nach einem oder mehreren der vorhergehenden Ansprüche, **dadurch gekennzeichnet, dass** die Notfall-Steuereinheit (5) mindestens eine Notfall-Eingabe-/Ausgabevorrichtung (13) umfasst, die dazu ausgebildet ist, das biomedizinische Gerät (4) zu kontrollieren/überwachen, wobei die Notfall-Eingabe-/Ausgabevorrichtung (13) vom Typ einer Tastatur, eines Bildschirms, einer USB-Buchse, eines Code-Lesegeräts oder eines elektronischen Schalters ist.

5. Ausrüstung (1) nach einem oder mehreren der vorhergehenden Ansprüche, **dadurch gekennzeichnet, dass** sie Verbindungsmittel (16, 19) des biomedizinischen Geräts (4) zu entweder der Steuereinheit (3) oder der Notfall-Steuereinheit (5) umfasst, wobei die Verbindungsmittel (16, 19) in einer lösbaren Weise zwischen mindestens einer ersten Betriebskonfiguration, in der sie das biomedizinische Gerät (4) in Wirkverbindung mit der Steuereinheit (3) bringen, und mindestens einer zweiten Betriebskonfiguration, in der sie das biomedizinische Gerät (4) in Wirkverbindung mit der Notfall-Steuereinheit (5) bringen, verbindbar sind.

6. Ausrüstung (1) nach einem oder mehreren der vorhergehenden Ansprüche, **dadurch gekennzeichnet, dass** die Verbindungsmittel (16, 19) in Wirkverbindung mit einer Vielzahl von Komponenten (15, 22, 23) des biomedizinischen Geräts (4) gebracht werden können, wobei die Verbindungsmittel (16, 19) mindestens eine Komponente (15, 22, 23) in der ersten Betriebskonfiguration mit der Steuereinheit (3) und in der zweiten Betriebskonfiguration mit der Notfall-Steuereinheit (5) in lösbarer Weise verbinden.

7. System (14) für die Steuerung von biomedizinischen Geräten während des extrakorporalen Kreislaufs, umfassend:
- mindestens ein biomedizinisches Gerät (4) für den extrakorporalen Kreislauf;
- mindestens eine Ausrüstung (1) nach einem oder mehreren der Ansprüche 1 bis 6.

8. System (14) nach Anspruch 7, **dadurch gekennzeichnet, dass**:
- das biomedizinische Gerät (4) mindestens eine motorgetriebene Pumpeinheit (15) für den extrakorporalen Kreislauf umfasst,
- die Verbindungsmittel (16, 19) mindestens eine Verbindungsleitung (16) umfassen, die die motorgetriebene Einheit (15) in der ersten Betriebskonfiguration mit der Steuereinheit (3) und in der zweiten Betriebskonfiguration mit der Notfall-Steuereinheit (5) in Wirkverbindung bringt.

9. System (14) nach einem oder mehreren der Ansprüche 7 bis 8, **dadurch gekennzeichnet, dass**:
- das biomedizinische Gerät (4) mindestens eine Überwachungseinheit (22, 23) des extrakorporalen Kreislaufs umfasst, die mit mindestens einer Sensorvorrichtung (22) ausgestattet ist, die aus den Folgenden ausgewählt ist: Strömungsdrucksensor, Füllstandssensor, Inline-Luftdetektor, Venensonde, Arteriensonde;
- die Verbindungsmittel (16, 19) mindestens eine Verbindungsleitung (19) umfassen, die die Sensorvorrichtung (22) in der ersten Betriebskonfiguration mit der Steuereinheit (3) und in der zweiten Betriebskonfiguration mit der Notfall-Steuereinheit (5) in Wirkverbindung bringt.

10. System (14) nach Anspruch 9, **dadurch gekennzeichnet, dass** die Überwachungseinheit (22, 23) eine Vielzahl der Sensorvorrichtungen (22) umfasst.

11. System (14) nach Anspruch 9, **dadurch gekennzeichnet, dass**:
- die Überwachungseinheit (22, 23) mindestens eine Notfall-Sensorvorrichtung (23) umfasst, die aus den Folgenden ausgewählt ist: Durchflussmesser, Drucksensor, Füllstandsensor, Inline-Luftdetektor, Venensonde, Arteriensonde;
- die Verbindungsleitung (19) die Steuereinheit (3) in der ersten Betriebskonfiguration mit der Sensorvorrichtung (22) in Wirkverbindung bringt und die Notfall-Steuereinheit (5) in der zweiten Betriebskonfiguration entweder mit der Sensorvorrichtung (22) oder mit der Notfall-Sensorvorrichtung (23) in Wirkverbindung bringt.

## Revendications

1. - Équipement (1) pour la commande de dispositifs biomédicaux pendant une circulation extracorporelle, comprenant :
- au moins une structure de base (2) ;
- des moyens de commande (3, 5, 9, 10, 11) assemblés sur ladite structure de base (2) et comportant au moins une unité de commande (3) apte à être connectée de manière fonctionnelle à au moins un dispositif biomédical (4) pour une circulation extracorporelle et configurée pour le commander ;
dans lequel lesdits moyens de commande (3, 5, 9, 10, 11) comprennent au moins une unité de commande d'urgence (5), apte à être connectée de manière fonctionnelle audit dispositif biomédical (4) et configurée pour commander ce dernier suite à un dysfonctionnement de ladite unité de commande (3) ;
dans lequel lesdits moyens de commande (3, 5, 9, 10, 11) comprennent des moyens de communication (9) entre ladite unité de commande (3) et ladite unité de commande d'urgence (5), ladite unité de commande d'urgence (5) étant configurée pour vérifier le dysfonctionnement de ladite unité de commande (3) et
**caractérisé par le fait que** ladite unité de commande (3) est configurée pour vérifier le dysfonctionnement de ladite unité de commande d'urgence (5) pendant le fonctionnement correct de ladite unité de commande (3).

2. - Équipement (1) selon la revendication 1, **caractérisé par le fait que** ladite structure de base (2) comprend des moyens de support (6) dudit dispositif biomédical (4) aptes à attacher ce dernier à la même structure de base de manière amovible.

3. - Équipement (1) selon une ou plusieurs des revendications précédentes, **caractérisé par le fait que** lesdits moyens de commande (3, 5, 9, 10, 11) comprennent au moins un dispositif d'entrée/sortie (12) configuré pour vérifier/surveiller ledit dispositif biomédical (4), ledit dispositif d'entrée/sortie (12) étant du type de ceux choisis dans la liste comprenant un clavier, un écran, une prise USB, un lecteur de code, un commutateur électronique.

4. - Équipement (1) selon une ou plusieurs des revendications précédentes, **caractérisé par le fait que** ladite unité de commande d'urgence (5) comprend au moins un dispositif d'entrée/sortie d'urgence (13) configuré pour vérifier/surveiller ledit dispositif biomédical (4), ledit dispositif d'entrée/sortie d'urgence (13) étant du type de ceux choisis dans la liste comprenant un clavier, un écran, une prise USB, un lecteur de code, un commutateur électronique.

5. - Équipement (1) selon une ou plusieurs des revendications précédentes, **caractérisé par le fait qu'**il comprend des moyens de connexion (16, 19) dudit dispositif biomédical (4) à l'une de ladite unité de commande (3) et de ladite unité de commande d'urgence (5), lesdits moyens de connexion (16, 19) pouvant être connectés de manière amovible entre au moins une première configuration de fonctionnement, dans laquelle ils connectent de manière fonctionnelle ledit dispositif biomédical (4) à ladite unité de commande (3), et au moins une seconde configuration de fonctionnement, dans laquelle ils connectent de manière fonctionnelle ledit dispositif biomédical (4) à ladite unité de commande d'urgence (5).

6. - Équipement (1) selon une ou plusieurs des revendications précédentes, **caractérisé par le fait que** lesdits moyens de connexion (16, 19) sont aptes à être connectés de manière fonctionnelle à une pluralité de composants (15, 22, 23) dudit dispositif biomédical (4), lesdits moyens de connexion (16, 19) connectant de manière amovible au moins un composant (15, 22, 23) à ladite unité de commande (3) et à ladite unité de commande d'urgence (5) respectivement dans ladite première et dans ladite seconde configuration de fonctionnement.

7. - Système (14) pour la commande de dispositifs biomédicaux pendant une circulation extracorporelle, comprenant :
- au moins un dispositif biomédical (4) pour une circulation extracorporelle ;
- au moins une pièce d'équipement (1) selon une ou plusieurs des revendications 1 à 6.

8. - Système (14) selon la revendication 7, **caractérisé par le fait que** :
- ledit dispositif biomédical (4) comprend au moins une unité de pompage (15) entraînée par moteur pour une circulation extracorporelle ;
- lesdits moyens de connexion (16, 19) comprennent au moins une ligne de connexion (16) qui connecte de manière fonctionnelle ladite unité (15) entraînée par moteur à ladite unité de commande (3) et à ladite unité de commande d'urgence (5) respectivement dans ladite première et dans ladite seconde configuration de fonctionnement.

9. - Système (14) selon une ou plusieurs des revendications 7 à 8, **caractérisé par le fait que** :
- ledit dispositif biomédical (4) comprend au moins une unité de surveillance (22, 23) de la circulation extracorporelle comportant au moins un dispositif capteur (22) choisi dans la liste comprenant un débitmètre, un capteur de pression, un capteur de niveau, un détecteur d'air en ligne, une sonde veineuse, une sonde artérielle ;
- lesdits moyens de connexion (16, 19) comprennent au moins une ligne de liaison (19) qui connecte de manière fonctionnelle le dispositif capteur (22) à ladite unité de commande (3) et à ladite unité de commande d'urgence (5) respectivement dans ladite première et dans ladite seconde configuration de fonctionnement.

10. - Système (14) selon la revendication 9, **caractérisé par le fait que** ladite unité de surveillance (22, 23) comprend une pluralité desdits dispositifs capteurs (22).

11. - Système (14) selon la revendication 9, **caractérisé par le fait que** :
- ladite unité de surveillance (22, 23) comprend au moins un dispositif capteur d'urgence (23) choisi dans la liste comprenant un débitmètre, un capteur de pression, un capteur de niveau, un détecteur d'air en ligne, une sonde veineuse, une sonde artérielle ;
- ladite ligne de liaison (19) connecte de manière fonctionnelle ladite unité de commande (3) audit dispositif capteur (22) dans ladite première configuration de fonctionnement, et connecte de manière fonctionnelle ladite unité de commande d'urgence (5) à l'un dudit dispositif capteur (22) ou dudit dispositif capteur d'urgence (23) dans ladite seconde configuration de fonctionnement.
